# EUROPEAN PATENT APPLICATION

(11) **EP 4 606 821 A1**
(43) Date of publication of application: **27.08.2025**
(21) Application number: 23867619.1
(22) Date of filing: 22.09.2023
(51) Int. Cl.: C07K 16/28, C12N 15/13, A61K 39/395, A61P 35/00

(54) **FC?RI BINDING PROTEIN**

(30) Priority: 23.09.2022 CN 202211165661
(71) Applicant: Longbio Pharma (Suzhou) Co., Ltd., Shanghai 201203 (CN)
(72) Inventor: MA, Haili, Shanghai 201203 (CN); LIU, Yunhua, Shanghai 201203 (CN); LIU, Heng, Shanghai 201203 (CN)
(74) Representative: AWA Denmark A/S
(86) International application number: PCT/CN2023/120533
(87) International publication number: WO 2024/061334

(57) **Abstract**

Provided in the present application is a binding protein. The binding protein contains an IgG Fc region or a fragment thereof, and can bind to FcεRI. Also provided in the present application are a nucleic acid molecule encoding the binding protein; a vector and a cell which contain the nucleic acid molecule; a pharmaceutical composition containing the binding protein; and a use of the binding protein in the preparation of a drug.

## Description

### Field of the Invention

The present application relates to the field of biomedicine, and particularly to an FcεRI binding protein.

### Background of the Invention

The neonatal Fc receptor (FcRn) belongs to a broad and functionally distinct family of MHC molecules. In contrast to classical MHC family members, FcRn exhibits little diversity and cannot present antigens. Instead, FcRn regulates the serum half-life of IgG and albumin through its ability to bind these two proteins with high affinity at low pH. The serum half-life of IgG is significantly longer than that of similarly sized globular proteins, including IgE, which does not bind to FcRn (approximately 21 days for IgG and approximately < 2 days for IgE). In addition, FcRn plays an important role in immunity at mucosal and systemic sites through its ability to influence the lifespan of IgG and its involvement in both innate and adaptive immune responses.

The IgE receptor FcεRI is involved in IgE-mediated activation of a wide range of immune cells, leading to a variety of immune diseases, such as allergic diseases. Blocking the binding of IgE to the FcεRI receptor with FcεRI binding proteins has a potential for treating immune diseases. In addition, recruitment of relevant immune cells by targeting the FcεRI receptor could also be used for the treatment of tumors. Therefore, FcεRI binding proteins have a variety of therapeutic potentials.

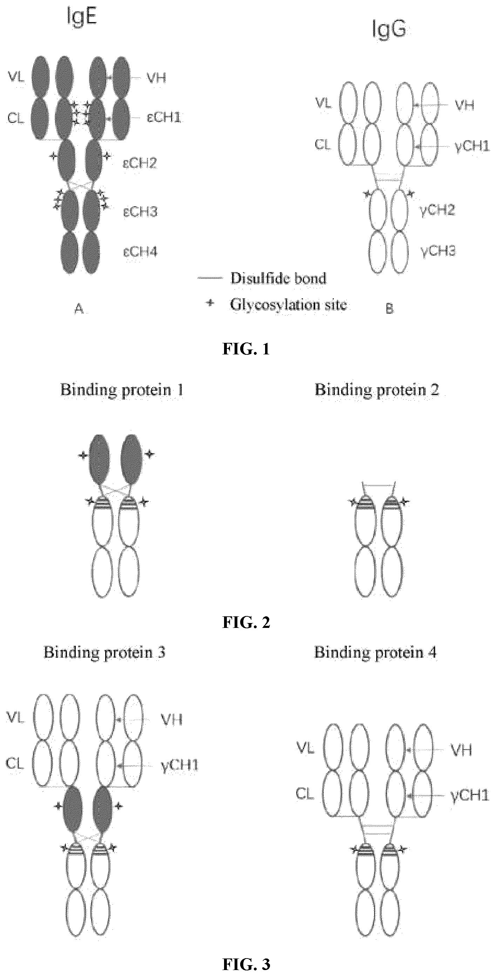

Natural IgE antibodies have strong FcεRI binding activity, but there are still many drawbacks for their development as drugs. For example, natural IgE has about 14 potential glycosylation sites (see Fig. 1A), which is not conducive to quality control after large-scale production; IgE Fc is unable to bind to Protein A or G and thus cannot be purified on large scale using the existing purification methods for well-established IgG-based drugs; IgE Fc does not have the ability to bind to FcRn, so the plasma half-life of natural IgE is short; in addition, the affinity of recombinant IgE for FcεRI is too high, 10-10 M (Metzger H. The receptor with high affinity for IgE. Immunol Rev. 1992; 125:37-48), which may cause severe allergic reactions when administered as a drug, among other things. In summary, there is a need for proteins with improved properties compared to both IgE and IgG.

### Summary of the Invention

The present application provides a binding protein, which has the ability to bind to FcεRIa while maintaining the good druggability properties associated with IgG Fc. The binding protein of the present application has one or more of the following properties:
(1) being capable of binding to FcεRIa; (2) being capable of binding to FcRn; (3) having good expression quality; (4) being capable of being purified directly with Protein A or G; and (5) having reduced potential glycosylation sites compared with IgE Fc.

In one aspect, the present application provides a binding protein capable of binding to FcεRI, the binding protein includes an IgG Fc region or a fragment thereof, the IgG Fc region or the fragment thereof includes BC-LOOP, DE-TURN and/or FG-LOOP or a fragment thereof derived from CH3 in an IgE Fc region, and regions in CH2 in the IgG Fc region corresponding to BC-LOOP, DE-TURN and/or FG-LOOP or the fragment thereof in CH3 in the IgE Fc region are substituted or replaced by BC-LOOP, DE-TURN and/or FG-LOOP or the fragment thereof in the CH3 in the IgE Fc region.

In some embodiments, the IgG is a human IgG.

In some embodiments, the IgG is IgG1, IgG2, IgG3 or IgG4.

In some embodiments, the CH2 in the wild-type IgG Fc region includes an amino acid sequence as set forth in any one of SEQ ID NO: 24, SEQ ID NO: 25, SEQ ID NO: 26, and SEQ ID NO: 27.

In some embodiments, the fragment of the IgG Fc region at least includes the CH2 in the IgG Fc region or a fragment thereof.

In some embodiments, the BC-LOOP, DE-TURN and/or FG-LOOP or the fragment thereof derived from the CH3 in the IgE Fc region is capable of binding to FcεRI.

In some embodiments, the BC-LOOP in the CH2 in the IgG Fc region includes an amino acid sequence of positions 27-38 in the CH2 in the IgG Fc region.

In some embodiments, the BC-LOOP in the CH2 in the IgG Fc region includes an amino acid sequence as set forth in SEQ ID NO: 1 or SEQ ID NO: 2.

In some embodiments, the DE-TURN in the CH2 in the IgG Fc region includes an amino acid sequence of positions 84.1-84.4 and 85.4-85.1 in the CH2 in the IgG Fc region.

In some embodiments, the DE-TURN in the CH2 in the IgG Fc region includes an amino acid sequence as set forth in any one of SEQ ID NO: 3, SEQ ID NO: 4, SEQ ID NO: 39, and SEQ ID NO: 40.

In some embodiments, the FG-LOOP in the CH2 in the IgG Fc region includes an amino acid sequence of positions 105-117 in the CH2 in the IgG Fc region.

In some embodiments, the FG-LOOP in the CH2 in the IgG Fc region includes an amino acid sequence as set forth in SEQ ID NO: 5, SEQ ID NO: 6 or SEQ ID NO: 7.

In some embodiments, the BC-LOOP in the CH3 in the IgE Fc region includes an amino acid sequence of positions 27-38 in the CH3 in the IgE Fc region.

In some embodiments, the BC-LOOP in the CH3 in the IgE Fc region includes an amino acid sequence as set forth in SEQ ID NO: 8.

In some embodiments, the DE-TURN in the CH3 in the IgE Fc region includes an amino acid sequence of positions 84.1-84.4 and 85.4-85.1 in the CH3 in the IgE Fc region, the DE-TURN in the CH3 in the IgE Fc region includes an amino acid sequence of positions 84.1-84.4 and 85.4-85.1 in the CH3 in the IgE Fc region.

In some embodiments, the DE-TURN in the CH3 in the IgE Fc region includes an amino acid sequence as set forth in SEQ ID NO: 9.

In some embodiments, the FG-LOOP in the CH3 in the IgE Fc region includes an amino acid sequence of positions 105-117 in the CH3 in the IgE Fc region.

In some embodiments, the FG-LOOP in the CH3 in the IgE Fc region includes an amino acid sequence as set forth in SEQ ID NO: 10.

In some embodiments, a region location of the amino acid is determined by means of IMGT unique numbering for C-DOMAIN coding.

In some embodiments, the CH2 in the IgG Fc region includes an amino acid sequence as set forth in SEQ ID NO: 11 or SEQ ID NO: 41.

In another aspect, the present application provides a binding protein comprising an IgG Fc region or a fragment thereof. Compared with a wild-type IgG Fc region, the IgG Fc region or the fragment thereof includes one or more amino acid mutations, and the mutated IgG Fc region or the fragment thereof is capable of binding to FcεRI.

In some embodiments, the IgG includes a human IgG.

In some embodiments, the IgG includes IgG1, IgG2, IgG3 or IgG4.

In some embodiments, the CH2 of the wild-type IgG Fc region includes an amino acid sequence as set forth in any one of SEQ ID NO: 24, SEQ ID NO: 25, SEQ ID NO: 26, and SEQ ID NO: 27.

In some embodiments, the IgG Fc region at least includes a CH2 in the IgG Fc region.

In some embodiments, the BC-LOOP in the CH2 in the IgG Fc region has one or more amino acid mutations.

In some embodiments, the BC-LOOP in the CH2 in the IgG Fc region has amino acid mutations at one or more sites selected from a group consisting of: V28, S29, H30, E31, D34, P35 and/or K/Q38.

In some embodiments, the BC-LOOP in the CH2 in the IgG Fc region has one or more amino acid mutations selected from a group consisting of: V28L, S29A, H30P, E31S, D34K, P35G, E36T, and/or K/Q38N.

In some embodiments, the BC-LOOP in the CH2 in the mutated IgG Fc region includes an amino acid sequence as set forth in SEQ ID NO: 8.

In some embodiments, the DE-TURN in the CH2 in the IgG Fc region has amino acid mutations at one or more sites selected from a group consisting of: E84.1, Y/F84.3, S85.4, Y/F85.2, and/or R85.1.

In some embodiments, the DE-TURN in the CH2 in the IgG Fc region has one or more amino acid mutations selected from a group consisting of: E84.1K, Y/F84.3R, S85.4G, Y/F85.2L, and/or R85.1T.

In some embodiments, the DE-TURN in the CH2 in the mutated IgG Fc region includes an amino acid sequence as set forth in SEQ ID NO: 9.

In some embodiments, the FG-LOOP in the CH2 in the IgG Fc region has amino acid mutations at one or more sites selected from a group consisting of: K105, S107, N108, K109, A/G110, A/S115, P/S116, and/or I117.

In some embodiments, the FG-LOOP in the CH2 in the IgG Fc region has one or more amino acid mutations selected from a group consisting of: K105R, S107T, N108H, K109P, A/G110H, A/S115R, P/S116A, and/or I117L.

In some embodiments, the FG-LOOP in the CH2 in the mutated IgG Fc region includes an amino acid sequence as set forth in SEQ ID NO: 10.

In some embodiments, the IgG Fc region includes an amino acid sequence as set forth in SEQ ID NO: 11, SEQ ID NO: 38 or SEQ ID NO: 41.

In some embodiments, an N terminus of the CH2 in the IgG Fc region has amino acid mutations at one or more sites selected from a group consisting of: P1.5, E1.4, L/P/F1.3, L/V1.2, G/A1.1, G1, and/or P2.

In some embodiments, the N terminus of the CH2 in the IgG Fc region has one or more amino acid mutations selected from a group consisting of: P1.5D, E1.4S, L/P/F1.3N, L/V1.2P, G/A1.1R, and/or P2V.

In some embodiments, a region location of the amino acid is determined by means of IMGT unique numbering for C-DOMAIN coding.

In some embodiments, the CH2 in the mutated IgG Fc region includes an amino acid sequence as set forth in SEQ ID NO: 11 or SEQ ID NO: 41.

In some embodiments, the binding protein further includes a CH3 in the IgG Fc region.

In some embodiments, the binding protein includes an amino acid sequence as set forth in SEQ ID NO: 29 or SEQ ID NO: 38.

In some embodiments, the binding protein further includes a CH2 in a constant region of IgE or a fragment thereof.

In some embodiments, the IgG Fc region of the binding protein includes an amino acid sequence as set forth in any one of SEQ ID NO: 16, SEQ ID NO: 17, SEQ ID NO: 18, SEQ ID NO: 19, SEQ ID NO: 30, and SEQ ID NO: 31.

In some embodiments, the binding protein comprises an amino acid sequence as set forth in any one of SEQ ID NO: 16, SEQ ID NO: 17, SEQ ID NO: 18, SEQ ID NO: 19, SEQ ID NO: 30, and SEQ ID NO: 31.

In some embodiments, the binding protein has one or more of the following properties:
(1) being capable of binding to FcεRIa;
(2) being capable of binding to FcRn;
(3) having good expression quality;
(4) being capable of being purified directly with Protein A or G; and
(5) having reduced potential glycosylation sites compared with IgE Fc.

In another aspect, the present application provides a polypeptide including the binding protein. In another aspect, the present application provides a fusion protein including the binding protein. In some embodiments, the fusion protein includes one or more other functionally active proteins.

In some embodiments, the other functionally active proteins are one or more selected from a group consisting of: Fab, scFv, VHH, cytokines, soluble receptors or ligands, and pharmaceutical polypeptides.

In some embodiments, the fusion protein is an IgG-like molecule.

In some embodiments, the fusion protein is capable of specifically binding to a tumor-associated antigen.

In some embodiments, the tumor-associated antigen includes Her2.

In some embodiments, the heavy chain of the IgG-like molecule includes an amino acid sequence as set forth in SEQ ID NO: 20 or SEQ ID NO: 21.

In some embodiments, the light chain of the IgG-like molecule includes an amino acid sequence as set forth in SEQ ID NO: 22.

In some embodiments, the tumor-associated antigen includes GPC3.

In some embodiments, the heavy chain of the IgG-like molecule includes an amino acid sequence as set forth in SEQ ID NO: 32 or SEQ ID NO: 33.

In some embodiments, the light chain of the IgG-like molecule includes an amino acid sequence as set forth in SEQ ID NO: 34.

In some embodiments, the tumor-associated antigen includes CD20.

In some embodiments, the heavy chain of the IgG-like molecule includes an amino acid sequence as set forth in SEQ ID NO: 35 or SEQ ID NO: 36.

In some embodiments, the light chain of the IgG-like molecule includes an amino acid sequence as set forth in SEQ ID NO: 37.

In another aspect, the present application also provides a drug molecule including the binding protein, the polypeptide or the fusion protein.

In another aspect, the present application also provides one or more isolated nucleic acid molecules encoding the binding protein, the polypeptide or the fusion protein.

In another aspect, the present application also provides a vector including the nucleic acid molecule.

In another aspect, the present application also provides a cell including the nucleic acid molecule or the vector.

In another aspect, the present application also provides a pharmaceutical composition including the binding protein, the polypeptide, the fusion protein, the drug molecule, the nucleic acid molecule, the vector or the cell, and optionally a pharmaceutically acceptable carrier.

In another aspect, the present application also provides a method for preparing the binding protein, the polypeptide, or the fusion protein, the method includes culturing the cell under a condition enabling the expression of the binding protein, the polypeptide or the fusion protein.

In another aspect, the present application also provides use of the binding protein, the polypeptide, the fusion protein, the drug molecule, the nucleic acid molecule, the vector, the cell or the pharmaceutical composition in preparation of a drug for preventing or treating a disease and/or disorder.

In some embodiments, the disease and/or disorder includes a tumor.

In some embodiments, the tumor includes a solid tumor and/or a blood tumor.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### Brief Description of the Drawings

The specific features of the invention involved in the present application are shown in the appended claims. The characteristics and advantages of the invention involved in the present application can be better understood by referring to the exemplary embodiments and the accompanying drawings described in detail below. A brief description of the drawings is as follows:
FIG. 1 shows the structures of IgE and IgG and a schematic diagram of potential glycosylation modification sites.
FIG. 2 shows the structures of binding proteins and a schematic diagram of potential glycosylation modification sites.
FIG. 3 shows the structures of binding proteins fused with Fab and a schematic diagram of potential glycosylation modification sites.
FIG. 4A shows the IMGT coding and regional division of the constant region CH3 of a heavy chain of human IgE; FIG. 4B shows the IMGT coding and regional division of the constant region CH2 of a heavy chain of human IgG; and the coding mode is IMGT unique numbering for C-DOMAIN.

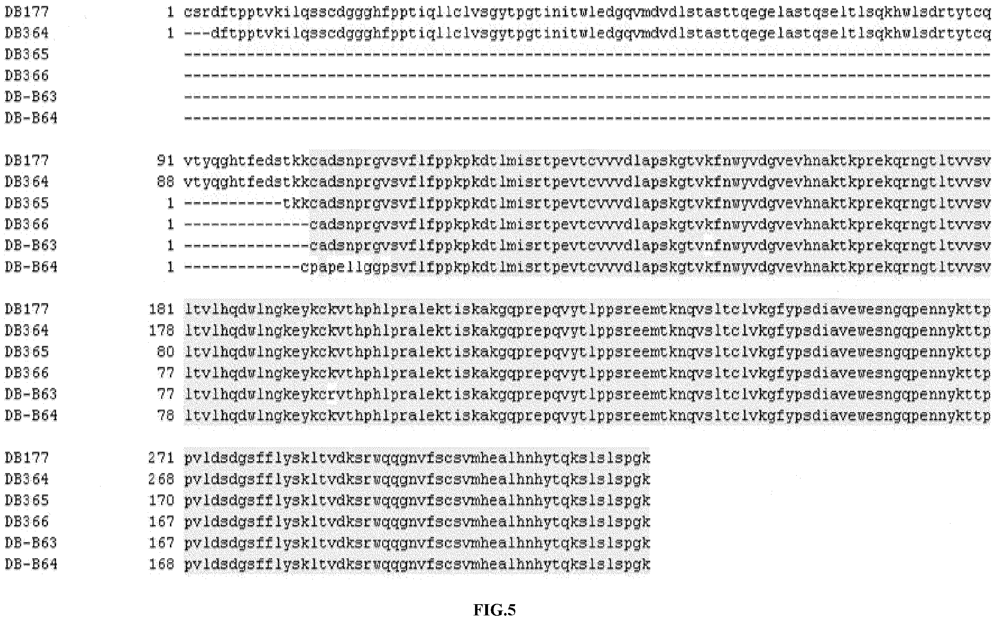

FIG. 5 shows the amino acid sequences of different binding proteins and the sequence alignment results.
FIG. 6 shows the affinity assay results of different binding proteins for recombinant human FcεRIa.
FIG. 7 shows a diagram of SEC-HPLC detection results of binding proteins DB 177, DB364, and DB365.
FIG. 8 shows a diagram of SEC-HPLC detection results of binding proteins DB366, DB-B63, and DB-B64.
FIG. 9 shows a diagram of SEC-HPLC detection results of Her2-specific binding proteins DB871 and DB872.
FIG. 10 shows a diagram of SEC-HPLC detection results of GPC3-specific binding proteins DB-B229 and DB-B230.
FIG. 11 shows a diagram of SEC-HPLC detection results of CD20-specific binding proteins DB-B232 and DB-B233.
FIGs. 12A-12C show the ELISA detection results of the blocking activity of the binding proteins of the present application to block the binding of human IgE to the receptor FcεRIa.

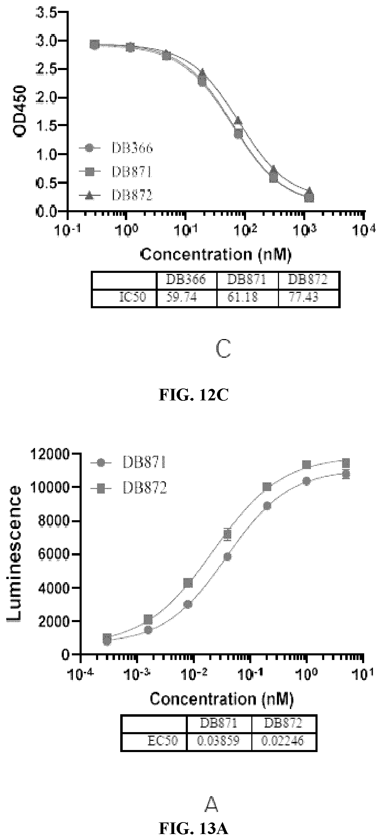

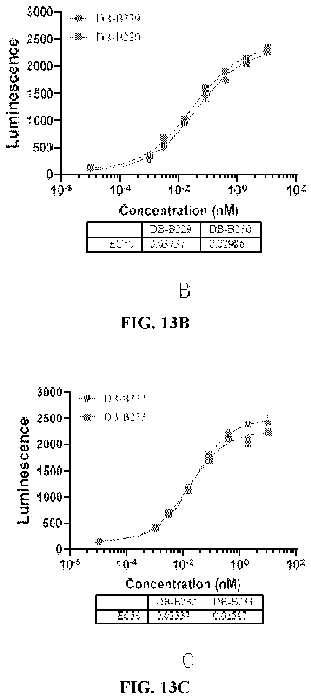

FIGs. 13A-13C show the detection results of the activity of the binding protein-mediated activated mastocytes to express luciferase reporter genes. FIG. 13A shows the detection results of the activity of Her2-specific binding proteins DB871 and DB872-mediated NCI-N87 cell-activated mastocytes to express luciferase reporter genes; FIG. 13B shows the detection results of the activity of GPC3-specific binding protein DB-B229 and DB-B230-mediated HepG2 cell-activated mastocytes to express luciferase reporter genes; FIG. 13C shows the detection results of the activity of CD20-specific binding proteins DB-B232 and DB-B233-mediated Raji cell-activated mastocytes to express luciferase reporter genes.

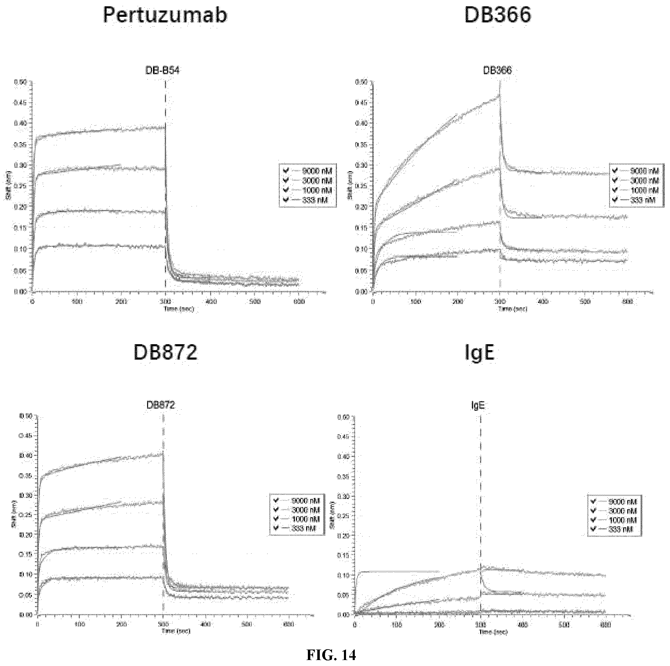

FIG. 14 shows the detection results of the binding of a control antibody and binding proteins to recombinant human FcRn.

### Detailed Description of the Embodiments

The implementation of the present application will be illustrated in the following specific examples, and other advantages and effects of the present application will be easily known by those skilled in the art from the content disclosed in the specification.

### Definition of Terms

In the present application, the term "amino acid mutation" generally encompasses amino acid substitution, deletion, insertion, and modification. Any combination of substitution, deletion, insertion, and modification can be made to implement the final construct, as long as the final construct possesses the desired properties. A particular amino acid mutation is amino acid substitution. For example, in order to change the binding properties of, for example, the Fc region, non-conservative amino acid substitution can be selected, i.e., replacing one amino acid with another amino acid having different structural and/or chemical properties. Amino acid substitutions include replacement by non-naturally occurring amino acids or by naturally occurring amino acid derivatives of the 20 standard amino acids (e.g., 4-hydroxyproline, 3-methylhistidine, ornithine, homoserine, 5-hydroxylysine). Amino acid mutations can be generated using genetic or chemical methods well known in the art. Genetic methods may include site-directed mutagenesis, PCR, gene synthesis, etc. Methods of altering amino acid side chain groups by methods other than genetic engineering, such as chemical modification, are also available.

In the present application, "Xn" refers to a residue X corresponding to position n in the amino acid sequence of the wild-type IgG Fc region, where n is a positive integer, X is abbreviation of any amino acid residue, and the location of the sequence is determined by an IMGT unique numbering for C-DOMAIN numbering scheme which is a commonly used numbering scheme known to those skilled in the art. For example, "K105" represents the amino acid K at position 105 corresponding to the sequence of CH2 in the IgG Fc region by using the IMGT unique numbering for C-DOMAIN numbering scheme.

In the present application, an amino acid substitution of "X/YnZ" means that according to the numbering scheme in the present application, the residue X or Y at position n in the amino acid sequence is substituted for an amino acid residue Z, where n is a positive integer, and X, Y, and Z are independently abbreviations of any amino acid residues, respectively.

In the present application, the terms "BC-LOOP", "DE-TURN", "FG-LOOP" generally refer to a segment of amino acid sequence of the Fc region of an immunoglobulin molecule, which can be determined by means of IMGT unique numbering for C-DOMAIN coding. Such definitions are apparent to those skilled in the art. For example, the BC-LOOP in the CH2 in the IgG Fc region includes an amino acid sequence of positions 27-38 in the CH2 in the IgG Fc region. For example, the DE-TURN in the CH2 in the IgG Fc region includes an amino acid sequence of positions 84.1-84.4 and 85.4-85.1 in the CH2 in the IgG Fc region. For example, the FG-LOOP in the CH2 in the IgG Fc region includes an amino acid sequence of positions 105-117 in the CH2 in the IgG Fc region. For example, the BC-LOOP in the CH3 in the IgE Fc region includes an amino acid sequence of positions 27-38 in the CH3 in the IgE Fc region. For example, the DE-TURN in the CH3 in the IgE Fc region includes an amino acid sequence of positions 84.1-84.4 and 85.4-85.1 in the CH3 in the IgE Fc region. For example, the FG-LOOP in the CH3 in the IgE Fc region includes an amino acid sequence of positions 105-117 in the CH3 in the IgE Fc region.

In the present application, the term "polypeptide" generally refers to a polymer of amino acid residues. This term also applies to amino acid polymers in which one or more amino acid residues are analogues or mimics of corresponding naturally occurring amino acids, and to naturally occurring amino acid polymers. This term may also include amino acid polymers that are modified, for example, by the addition of sugar residues to form glycoproteins or by phosphorylation. The polypeptide may be produced from naturally occurring and non-recombinant cells or from genetically engineered or recombinant cells, and may include a molecule having the amino acid sequence of a natural protein, or a molecule having deletions, additions and/or substitutions of one or more amino acids of the natural sequence. The term "polypeptide" may include an antigen-binding fragment, an antibody or a sequence having deletions, additions and/or substitutions of one or more amino acids of the antigen-binding fragment. The term "polypeptide" may refer to a polypeptide having an amino-terminal deletion, a carboxy-terminal deletion and/or an internal deletion as compared to a full-length protein. Such fragments may also contain modified amino acids compared to full-length proteins.

In the present application, the term "VHH" generally refers to an antibody containing a variable antigen-binding domain of a heavy chain antibody. VHH may also be referred to as Nanobody (Nb) and/or a single-domain antibody.

The term "Fab" refers to an antibody fragment composed of a complete L chain ((V_{L} and C_{L}) together with a variable region domain (VH) of an H chain and a first constant domain (C_{H}1) of a heavy chain. Papain digestion of intact antibodies can be used to generate two Fab fragments, each of which contains a single antigen-binding site. In general, the L- and H-chain fragments of Fab generated by papain digestion are linked by an interchain disulfide bond.

In the present application, the term "scFv" generally refers to VL and VH linked together using a recombinant methods or by a synthetic linker, where the synthetic linker can produce them as a single protein chain, where the VL and VH regions are paired to form a monovalent molecule (referred to as single chain Fv (scFv); see, for example, Bird et, al., Science 242:423-426 (1988), and Huston et, al., Proc. Natl. Acad. Sci USA 85:5879-5883 (1988)).

In the present application, the term "FcεRI" generally refers to a high affinity receptor for the Fc region of immunoglobulin E (IgE) (FcεRI, also referred to as Fc epsilon RI). FcεRI is a tetramer receptor complex capable of binding to the Fc region of ε heavy chain of IgE, which is composed of one α-chain (i.e., FcεRIα), one β-chain (FcεRIβ), and two γ-chains (FcεRIγ). Generally, the α-chain can act as a binding site for antibodies (e.g. IgE), the γ-chain can act as a site for downstream signal initiation, and the β-chain can act to amplify downstream signals. FcεRI is expressed on epidermal Langerhans cells, eosinophils, mastocytes, and basophils. Due to its cellular distribution, this receptor plays a major role in allergic reactions. FcεRI may be also expressed on antigen-presenting cells, and is involved in the production of important immune mediators that promote inflammation (e.g., cytokines, interleukins, leukotrienes, and prostaglandins, etc.).

In the present application, the term "specifically binding to" or "specific" generally refers to measurable and reproducible interactions, such as the binding between targets and antibodies, which can determine the presence of the targets in the presence of a heterogeneous population of molecules, including biomolecules. For example, an antibody specifically binding to a target (which may be an epitope) is an antibody that binds to the target with greater affinity, avidity, easier, and/or for a greater duration than it binds to other targets. In some embodiments, the antibody specifically binds to the epitope on the protein, and the epitope is conservative among proteins of different species. In some embodiments, specific binding may include, but does not require exclusive binding.

In the present application, the term "nucleic acid" generally refers to a polymer of nucleotides (e.g., ribonucleotide or deoxyribonucleotide), and may include naturally occurring (adenine, guanine, cytosine, uracil, and thymidine), non-naturally occurring, and modified nucleic acids. The term "nucleic acid" may include a gene, cDNA or mRNA. For example, the nucleic acid molecule may be synthesized (e.g., chemically synthesized) or recombinant. The nucleic acid may include a nucleic acid containing analogs or derivatives of naturally occurring nucleotides, which has similar binding properties to those of the reference nucleic acid and is metabolized in a manner similar to naturally occurring nucleotides. The nucleic acid sequence may also include conservative modified variants thereof (for example, degenerate codon substituted), alleles, orthologs, SNPs, and complementary sequences as well as the sequences explicitly indicated. The term is not limited by the length of the polymer. The nucleic acid may be single or double stranded and generally contain 5'-3' phosphodiester bonds, and nucleotide analogs may have other linkages.

In the present application, the term "cell" generally refers to an individual cell, cell line, or cell culture that may include or has included a plasmid or vector containing the nucleic acid molecule of the present application, or that can express the fusion protein of the present application or the antigen-binding fragment thereof. The cells may include the progeny of a single cell. Due to natural, accidental, or intentional mutations, the progeny cells may not necessarily be exactly the same as the original parent cells in terms of morphology or genome, as long as they can express the fusion protein of the present application or the antigen-binding fragment thereof. The cells can be obtained by transfecting cells with the vector of the present application *in vitro.* The cells may be prokaryotic cells (e.g., E. coli), or eukaryotic cells (e.g., yeast cells, such as COS cells, Chinese Hamster Ovary (CHO) cells, HeLa cells, HEK293 cells, COS-1 cells, or myeloma cells).

In the present application, the term "vector" generally refers to a nucleic acid molecule that is capable of self-replicating in a suitable host and that transfers the inserted nucleic acid molecule into and/or between cells (e.g., host cells). The vector may include vectors primarily for inserting DNA or RNA into cells, vectors primarily for replicating DNA or RNA, as well as vectors primarily for expression by transcription and/or translation of DNA or RNA. The vector also includes vectors having a plurality of the above functions. The vector may be a polynucleotide that can be transcribed and translated into a polypeptide when introduced into a suitable cell. Typically, the vector can produce the desired expression product by culturing suitable cells containing the vector. In the present application, the vector may be a plasmid.

In the present application, the term "pharmaceutical composition" generally refers to a composition suitable for administering to patients that may be human patients. For example, the pharmaceutical composition of the present application may include the antigen-binding protein of the present application, the immunoconjugate of the present application, the nucleic acid molecule of the present application, the vector of the present application and/or the cell of the present application, and optionally a pharmaceutically acceptable carrier. In addition, the pharmaceutical composition may also include one or more (pharmaceutically effective) carriers, stabilizers, excipients, diluents, solubilizers, surfactants, emulsifiers and/or preservatives, and other suitable preparations. The acceptable ingredients of the composition may be non-toxic to the recipient at the dosage and concentration used. The pharmaceutical composition of the present application may include, but not limited to, liquid, frozen and freeze-dried compositions.

In the present application, the term "subject" generally refers to human or non-human animals, including but not limited to, cat, dog, horse, pig, cow, sheep, rabbit, mouse, rat, or monkey.

In the present application, the term "IgG" generally refers to Immunoglobulin G. IgG is one of human immunoglobulins, others including lgA, lgM, IgD, and lgE. There are four subtypes of human IgG based on the differences in the antigenicity of the γ chain in the IgG molecule: IgG1, IgG2, IgG3, and IgG4. In the present application, the term "IgG1" generally refers to the most abundant subtype of IgG, with a high affinity for the Fc receptor. For example, the IgG may be a human IgG. Further for example, the IgG may be selected from a group consisting of: IgG1, IgG2, IgG3, and IgG4.

In the present application, the term "IgG-like molecule" generally refers to a molecule with a structure similar to that of an IgG-like molecule. For example, the IgG-like molecule may include a constant region and a variable region. For example, the IgG-like molecule may include a light chain and a heavy chain. The IgG-like molecule basically includes various domains of the IgG molecule (not limited to the target), but each domain may be modified to make the IgG-like molecule have a new function or make its activity be changed.

In the present application, the term "fusion protein" generally refers to a chimeric protein containing amino acid sequences of two or more different proteins. Typically, the fusion protein can be produced by an *in vitro* recombination strategy well known in the art.

In the present application, the term "drug molecule" generally refers to a molecule having the desired biological efficacy. The drug may be preventative or therapeutic. The drug molecule may include, without limitation: protein molecules, including, but not limited to, peptides, polypeptides, proteins, including post-translationally modified proteins, fusion proteins, antibodies, and the like; small molecules, including inorganic or organic compounds; and nucleic acid molecules, including, but not limited to, double-stranded or single-stranded DNA, or double-stranded or single-stranded RNA (e.g., antisense (molecules), RNAi, and the like), intron sequences, triple helical nucleic acid molecules, and aptamers; or vaccines.

The protein, polypeptide and/or amino acid sequences involved in the present application should also be understood to include at least the following ranges: variants or homologs having the same or similar functions as those of the protein or polypeptide.

In the present application, the variants may be, e.g., proteins or polypeptides with one or more amino acid substitutions, deletions or additions in the amino acid sequence of the protein and/or the polypeptide. For example, the functional variants may include proteins or polypeptides with amino acid changes by at least 1, for example, 1-30, 1-20 or 1-10, and further for example, 1, 2, 3, 4 or 5 amino acid substitutions, deletions and/or insertions. The functional variants can substantially maintain the biological properties of the protein or the polypeptide before change (e.g., substitution, deletion or addition). For example, the functional variants can maintain at least 60%, 70%, 80%, 90%, or 100% of the biological activity (e.g., antigen binding ability) of the protein or the polypeptide before change. For example, the substitution may be conservative substitution.

In the present application, the homologs may be proteins or polypeptides having at least about 85% (e.g., at least about 85%, about 90%, about 91%, about 92%, about 93%, about 94%, about 95%, about 96%, about 97%, about 98%, about 99% or higher) sequence homology with the amino acid sequence of the protein, and/or the polypeptide.

In the present application, the homology generally refers to the similarity, likeness or correlation between two or more sequences. The "percentage of sequence homology" can be calculated by: comparing two sequences to be aligned in a comparison window to determine the number of positions where the same nucleic acid bases (e.g., A, T, C, G, I) or the same amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are present in both sequences so as to obtain the number of matching positions, dividing the number of matching positions by the total number of positions in the comparison window (i.e., window size), and multiplying the result by 100, to generate the percentage of sequence homology. The alignment for determining the percentage of sequence homology can be achieved in a variety of ways known in the art, for example, by using publicly available computer software, such as BLAST, BLAST-2, ALIGN or Megalign (DNASTAR) software. A person skilled in the art can determine appropriate parameters for aligning sequences, including any algorithms needed to achieve the maximum alignment over the full-length sequence range being compared or within the target sequence region. The homology can also be determined by the following methods: FASTA and BLAST. A description of FASTA algorithm can be found in "Improved tools for biological sequence comparison" to W. R. Pearson and D. J. Lipman, Proc. Natl. Acad. Sci., 85: 2444-2448, 1988; and "Rapid and Sensitive Protein Similarity Searches" to D. J. Lipman and W. R. Pearson, Science, 227: 1435-1441, 1989. A description of BLAST algorithm can be found in "Basic Local Alignment Search Tool" to S. Altschul, W. Gish, W. Miller, E. W. Myers and D. Lipman, Journal of Molecular Biology, 215: 403-410, 1990.

In the present application, the term "comprise/include" generally means the inclusion of the features expressly designated, but not excluding other elements.

In the present application, the term "about" generally refers to varying in a range of 0.5%-10% above or below a specified value, for example, varying in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% above or below a specified value.

### Detailed Description of the Invention

The binding protein involved in the present application can be obtained by transplanting the amino acids located in the BC-LOOP, DE-TURN and FG-LOOP regions of the IgE Fc CH3 domain to replace the corresponding amino acids in the BC-LOOP, DE-TURN and FG-LOOP regions of the IgG Fc CH2 domain (the above regions and amino acid locations refer to the IMGT unique coding for an antibody constant region, i.e., IMGT unique numbering for C-DOMAIN, and strand, turn and loop range division, i.e., Range of strand, turn and loop lengths in C-DOMAIN and C-LIKE-DOMAIN, https://www.imgt.org/). Modified IgG Fc CH2 was obtained by transplant replacement. The binding protein of the present application is capable of binding to FcεRIa.

The binding protein involved in the present application can be obtained by transplanting part of the amino acids located in the BC-LOOP, DE-TURN and FG-LOOP regions of the IgE Fc CH3 domain to replace the corresponding amino acids in the corresponding BC-LOOP, DE-TURN and FG-LOOP regions of the IgG Fc CH2 domain. For example, modified IgG Fc CH2 was obtained by replacing the amino acids in the BC-LOOP, DE-TURN, and FG-LOOP regions in the IgG Fc CH2 domain with part of the amino acids in the corresponding BC-LOOP, DE-TURN, and FG-LOOP regions of the IgE Fc CH3 domain.

In the present application, the binding protein can also be mutated in transplant regions.

The binding protein involved in the present application can also be obtained by mutating the amino acids located in the BC-LOOP, DE-TURN, and FG-LOOP regions of the IgG Fc CH2 domain.

The binding protein of the present application has the ability to bind to FcεRIa while maintaining good druggability properties associated with IgG Fc: 1. good quality of expression, with the purity of monomers up to 90% or more after one-step purification; 2. removal of excessive glycosylation sites in natural IgE for easy quality control; 3. ease of purification, suitable for being purified directly using Protein A or G; 4. being capable of binding to FcRn, with a long half-life; 5. small molecular weight and good tissue permeability; 6. can be easily fused with other proteins to construct IgG-like molecules, such as Fab, scFv, VHH, cytokines, soluble receptors or ligands, polypeptides, etc.

In one aspect, the present application provides a binding protein capable of binding to FcεRI, and the binding protein includes an IgG Fc region or a fragment thereof, the IgG Fc region or the fragment thereof includes BC-LOOP, DE-TURN and/or FG-LOOP or a fragment thereof derived from CH3 in an IgE Fc region, and regions in CH2 in the IgG Fc region corresponding to BC-LOOP, DE-TURN and/or FG-LOOP or the fragment thereof in CH3 in the IgE Fc region are substituted or replaced by BC-LOOP, DE-TURN and/or FG-LOOP or the fragment thereof in the CH3 in the IgE Fc region.

In the present application, the IgG may be a human IgG.

In the present application, the IgG may be IgG1, IgG2, IgG3 or IgG4.

In the present application, the CH2 in the human IgG Fc region before transplant/replacement may include an amino acid sequence as set forth in any one of SEQ ID NO: 24, SEQ ID NO: 25, SEQ ID NO: 26, and SEQ ID NO: 27. In the present application, the sequences of CH2 in different IgG Fc regions may vary, but all can correspond to identified amino acids according to the numbering scheme described in the present application.

In the present application, the fragment of the IgG Fc region may at least include the CH2 in the IgG Fc region or a fragment thereof. In the present application, the main region of transplant replacement is concentrated on the CH2 of the IgG Fc region.

In the present application, the BC-LOOP, DE-TURN and/or FG-LOOP derived from CH3 in the IgE Fc region or a fragment thereof is capable of binding to FcεRI.

In the present application, the BC-LOOP in the CH2 in the IgG Fc region includes an amino acid sequence of positions 27-38 in the CH2 in the IgG Fc region. For example, the BC-LOOP in the CH2 in the IgG Fc region may include an amino acid sequence as set forth in DVSHEDPEV(K/Q) (SEQ ID NO: 1 or SEQ ID NO: 2).

In the present application, the DE-TURN in the CH2 in the IgG Fc region may include an amino acid sequence of positions 84.1-84.4 and 85.4-85.1 in the CH2 in the IgG Fc region. For example, the DE-TURN in the CH2 in the IgG Fc region may include an amino acid sequence as set forth in EQ(Y/F)NST(Y/F)R (SEQ ID NO: 3, SEQ ID NO: 4, SEQ ID NO: 39 or SEQ ID NO: 40).

In the present application, the FG-LOOP in the CH2 in the IgG Fc region includes an amino acid sequence of positions 105-117 in the CH2 in the IgG Fc region. For example, the FG-LOOP in the CH2 in the IgG Fc region may include an amino acid sequence as set forth in KVSNK(A/G)LP(A/S)(P/S)I (SEQ ID NO: 5, SEQ ID NO: 6 or SEQ ID NO: 7).

In the present application, the BC-LOOP, DE-TURN and/or FG-LOOP or parts thereof in the CH2 in the IgG Fc region can be replaced by the corresponding BC-LOOP, DE-TURN and/or FG-LOOP or parts thereof in the CH3 in the IgE Fc region.

In the present application, the BC-LOOP in the CH3 in the IgE Fc region may include an amino acid sequence of positions 27-38 in the CH3 in the IgE Fc region. For example, the BC-LOOP in the CH3 in the IgE Fc region may include an amino acid sequence as set forth in DLAPSKGTVN (SEQ ID NO: 8).

In the present application, the DE-TURN in the CH3 in the IgE Fc region may include an amino acid sequence of positions 84.1-84.4 and 85.4-85.1 in the CH3 in the IgE Fc region. For example, the DE-TURN in the CH3 in the IgE Fc region may include an amino acid sequence as set forth in KQRNGTLT (SEQ ID NO: 9).

In the present application, the FG-LOOP in the CH3 in the IgE Fc region may include an amino acid sequence of positions 105-117 in the CH3 in the IgE Fc region. For example, the FG-LOOP in the CH3 in the IgE Fc region may include an amino acid sequence as set forth in RVTHPHLPRAL (SEQ ID NO: 10).

In the present application, the CH2 in the IgG Fc region after transplant/replacement may include an amino acid sequence as set forth in SEQ ID NO: 11 or SEQ ID NO: 41.

In another aspect, the binding protein of the present application can be obtained from amino acid mutation on the CH2 fragment in the IgG Fc region. The binding protein provided in the present application includes an IgG Fc region or a fragment thereof, which, compared to a wild-type IgG Fc region, includes one or more amino acid mutations, and the mutated IgG Fc region or the fragment thereof can bind to FcεRI.

In the present application, the FcεRI may include FcεRIa.

In the present application, the IgG may include a human IgG.

In the present application, the IgG includes IgG1, IgG2, IgG3 or IgG4.

In the present application, the CH2 in the wild-type IgG Fc region may include an amino acid sequence as set forth in any one of SEQ ID NO: 24, SEQ ID NO: 25, SEQ ID NO: 26, and SEQ ID NO: 27.

In the present application, the IgG Fc region may at least include the CH2 in the IgG Fc region.

In the present application, the BC-LOOP in the CH2 in the IgG Fc region has one or more amino acid mutations.

In the present application, the BC-LOOP in the CH2 in the IgG Fc region may have amino acid mutations of V28, S29, H30, E31, D34, P35 and/or K/Q38. Where, K/Q38 means that, corresponding to different IgG Fc CH2 sequences, the amino acid at position 38 before the mutation can correspond to the amino acid K or Q.

In the present application, the BC-LOOP in the CH2 in the IgG Fc region may have amino acid mutations of V28L, S29A, H30P, E31S, D34K, P35G, E36T and/or K/Q38N.

In the present application, the BC-LOOP in the CH2 in the IgG Fc region may have amino acid mutations of V28L, S29A, H30P, E31S, D34K, P35G, E36T, and K/Q38N.

In the present application, the BC-LOOP in the CH2 in the IgG Fc region may have mutations listed in the table below:

| IMGT unique numbering for C-DOMAIN | 27 | 28 | 29 | 30 | 31 | 34 | 35 | 36 | 37 | 38 |
|---|---|---|---|---|---|---|---|---|---|---|
| Wild-type | D | V | S | H | E | D | P | E | V | K/Q |
| Mutant type | | L | A | P | S | K | G | T | | N |

In the present application, the BC-LOOP in the CH2 in the mutated IgG Fc region may include an amino acid sequence as set forth in SEQ ID NO: 8.

In the present application, the DE-TURN in the CH2 in the IgG Fc region may have amino acid mutations of E84.1, Y/F84.3, S85.4, Y/F85.2 and/or R85.1.

In the present application, the DE-TURN in the CH2 in the IgG Fc region may have amino acid mutations of E84.1, Y/F84.3, S85.4, Y/F85.2, and R85.1.

In the present application, the DE-TURN in the CH2 in the IgG Fc region may have amino acid mutations of E84.1K, Y/F84.3R, S85.4G, Y/F85.2L and/or R85.1T.

In the present application, the DE-TURN in the CH2 in the IgG Fc region may have amino acid mutations of E84.1K, Y/F84.3R, S85.4G, Y/F85.2L, and R85.1T.

In the present application, the DE-TURN in the CH2 in the IgG Fc region may have mutations listed in the table below:

| IMGT unique numbering for C-DOMAIN | 84.1 | 84.2 | 84.3 | 84.4 | 85.4 | 85.3 | 85.2 | 85.1 |
|---|---|---|---|---|---|---|---|---|
| Wild-type | E | Q | Y/F | N | S | T | Y/F | R |
| Mutant type | K | | R | | G | | L | T |

In the present application, the DE-TURN in the CH2 in the mutated IgG Fc region may include an amino acid sequence as set forth in SEQ ID NO: 9.

In the present application, the DE-TURN in the CH2 in the IgG Fc region may have amino acid mutations of E84.1K, Y/F84.3R, S85.4G, Y/F85.2L and/or R85.1T.

In the present application, the FG-LOOP in the CH2 in the IgG Fc region may have amino acid mutations of K105, S107, N108, K109, A/G110, A/S115, P/S116 and/or I117.

In the present application, the FG-LOOP in the CH2 in the IgG Fc region may have amino acid mutations of K105, S107, N108, K109, A/G110, A/S115, P/S116, and I117.

In the present application, the FG-LOOP in the CH2 in the IgG Fc region may have amino acid mutations of K105R, S107T, N108H, K109P, A/G110H, A/S115R, P/S116A and/or I117L.

In the present application, the FG-LOOP in the CH2 in the IgG Fc region may have amino acid mutations of K105R, S107T, N108H, K109P, A/G110H, A/S115R, P/S116A, and I117L.

In the present application, the FG-LOOP in the CH2 in the IgG Fc region may have mutations listed in the table below:

| IMGT unique numbering for C-DOMAIN | 105 | 106 | 107 | 108 | 109 | 110 | 113 | 114 | 115 | 116 | 117 |
|---|---|---|---|---|---|---|---|---|---|---|---|
| Wild-type | K | V | S | N/H | K | A/G | L | P | A/S | P/S | I |
| Mutant type | R | | T | H | P | H | | | R | A | L |

In the present application, the FG-LOOP in the CH2 in the mutated IgG Fc region may include an amino acid sequence as set forth in SEQ ID NO: 10.

In the present application, the N terminus of the CH2 in the IgG Fc region has amino acid mutations at P1.5, E1.4, L/P/F1.3, L/V1.2, G/A1.1, G1 and/or P2.

In the present application, the N terminus of the CH2 in the IgG Fc region may have amino acid mutations at P1.5, E1.4, L/P/F1.3, L/V1.2, G/A1.1, G1, and P2.

In the present application, the N terminus of the CH2 in the IgG Fc region may have amino acid mutations of P1.5D, E1.4S, L/P/F1.3N, L/V1.2P, G/A1.1R and/or P2V.

In the present application, the N terminus of the CH2 in the IgG Fc region may have amino acid mutations of P1.5D, E1.4S, L/P/F1.3N, L/V1.2P, G/A1.1R, and P2V.

In the present application, the N terminus of the CH2 in the IgG Fc region may have amino acid mutations listed in the table below:

| IMGT unique numbering for C-DOMAIN | 1.5 | 1.4 | 1.3 | 1.2 | 1.1 | 1 | 2 |
|---|---|---|---|---|---|---|---|
| Wild-type | P | E | L/P/F | L/V | G/A | G | P |
| Mutant type | D | S | N | P | R | G | V |

In the present application, the CH2 in the mutated IgG Fc region may include an amino acid sequence as set forth in SEQ ID NO: 11 or SEQ ID NO: 41.

In the present application, the binding protein may also include a CH3 in the IgG Fc region. For example, the binding protein may include an amino acid sequence as set forth in SEQ ID NO: 29 or SEQ ID NO: 38.

In the present application, the binding protein may also include a CH2 in the constant region of IgE or a fragment thereof.

In the present application, the IgG Fc region may include an amino acid sequence as set forth in any one of SEQ ID NO: 16, SEQ ID NO: 17, SEQ ID NO: 18, SEQ ID NO: 19, SEQ ID NO: 30, and SEQ ID NO: 31.

In another aspect, the present application also provides a polypeptide including the binding protein.

In another aspect, the present application also provides a fusion protein including the binding protein. In the present application, the fusion protein may also include one or more other functionally active molecules. For example, the other functionally active proteins may be one or more selected from a group consisting of: Fab, scFv, VHH, cytokines, soluble receptors or ligands, and pharmaceutical polypeptides.

For example, the fusion protein may be an IgG-like molecule. For example, the fusion protein may include the binding protein and a Fab molecule.

In the present application, the fusion protein is capable of specifically binding to a tumor-associated antigen.

For example, the tumor-associated antigen may include Her2. For example, the fusion protein may include a light chain and a heavy chain. For example, the light chain may include an amino acid sequence as set forth in SEQ ID NO: 22. For example, the heavy chain may include an amino acid sequence as set forth in SEQ ID NO: 20 or SEQ ID NO: 21. For example, the light chain may include an amino acid sequence as set forth in SEQ ID NO: 22 and the heavy chain may include an amino acid sequence as set forth in SEQ ID NO: 20. For example, the light chain may include an amino acid sequence as set forth in SEQ ID NO: 22 and the heavy chain may include an amino acid sequence as set forth in SEQ ID NO: 21.

For example, the tumor-associated antigen may include GPC3. For example, the fusion protein may include a light chain and a heavy chain. For example, the light chain may include an amino acid sequence as set forth in SEQ ID NO: 34. For example, the heavy chain may include an amino acid sequence as set forth in SEQ ID NO: 32 or SEQ ID NO: 33. For example, the light chain may include an amino acid sequence as set forth in SEQ ID NO: 34 and the heavy chain may include an amino acid sequence as set forth in SEQ ID NO: 32. For example, the light chain may include an amino acid sequence as set forth in SEQ ID NO: 34 and the heavy chain may include an amino acid sequence as set forth in SEQ ID NO: 33.

For example, the tumor-associated antigen may include CD20. For example, the fusion protein may include a light chain and a heavy chain. For example, the light chain may include an amino acid sequence as set forth in SEQ ID NO: 37. For example, the heavy chain may include an amino acid sequence as set forth in SEQ ID NO: 35 or SEQ ID NO: 36. For example, the light chain may include an amino acid sequence as set forth in SEQ ID NO: 37 and the heavy chain may include an amino acid sequence as set forth in SEQ ID NO: 35. For example, the light chain may include an amino acid sequence as set forth in SEQ ID NO: 37 and the heavy chain may include an amino acid sequence as set forth in SEQ ID NO: 36.

In another aspect, the present application provides a drug molecule, which includes the binding protein, the polypeptide or the fusion protein.

In another aspect, the present application provides one or more isolated nucleic acid molecules, which encode(s) the binding protein, the polypeptide or the fusion protein.

In another aspect, the present application provides one or more isolated nucleic acid molecule, which encode(s) the binding protein, the polypeptide or the fusion protein. The one or more isolated nucleic acid molecule of the present application may be isolated nucleotides, deoxyribonucleotides or ribonucleotides of any length, or analogs thereof isolated from the natural environment or synthesized artificially but capable of encoding the binding protein of the present application.

In another aspect, the present application also provides a vector, which may include the nucleic acid molecule of the present application. The vector can enable the expression of the genetic material elements it carries within a host cell by transforming, transducing or transfecting the host cell. For example, the vector may include plasmid; phagemid; Cosmid; artificial chromosomes, such as yeast artificial chromosomes (YACs), bacterial artificial chromosomes (BACs) or P1-derived artificial chromosomes (PACs); phages, such as lambda phages or M13 phages and animal viruses, and the like. The species of animal viruses used as the vector include retrovirus (including lentivirus), adenovirus, adeno-associated virus, herpes virus (e.g., herpes simplex virus), poxvirus, baculovirus, papilloma virus, papovavirus (e.g., SV40). Further for example, the vector may contain various elements for controlling the expression, including promoter sequences, transcription initiation sequences, enhancer sequences, selective elements and reporter genes. In addition, the vector may also contain replication initiation sites. Moreover, the vector may also include ingredients that help its entry into cells, such as virion, lipidosome or protein coat, but not only these substances.

In another aspect, the present application also provides a cell which may include the nucleic acid molecule of the present application or the vector of the present application. The cell may include the offsprings of a single cell. Due to natural, accidental or intentional mutations, the offsprings may not necessarily be exactly the same as the original parent cells (in terms of morphology of the total DNA complement or in terms of genome). In some embodiments, the cell may also include cells transfected with the vector of the present application *in vitro.* In some embodiments, the cells may be bacterial cells (e.g., *E. coli),* yeast cells, or other eukaryotic cells.

In another aspect, the present application also provides a pharmaceutical composition, which may include the binding protein of the present application, the polypeptide of the present application, the fusion protein of the present application, the nucleic acid molecule of the present application, the vector of the present application and/or the cell of the present application, and optionally a pharmaceutically acceptable carrier.

In some embodiments, the pharmaceutical composition may also include one or more (pharmaceutically effective) carriers, stabilizers, excipients, diluents, solubilizers, surfactants, emulsifiers and/or preservatives, and other suitable preparations. The acceptable ingredients of the composition may be preferably non-toxic to the recipient at the dosage and concentration used. The pharmaceutical composition of the present application includes, but not limited to, liquid, frozen and freeze-dried compositions.

In some embodiments, the pharmaceutically acceptable adjuvant may include any and all solvents, dispersion media, coatings, isotonic agents and absorption delaying agents that are compatible with the medication, which are generally safe, non-toxic and neither biologically nor otherwise undesirable.

In some embodiments, the pharmaceutical composition may be administered parenterally, transdermally, intraperitoneally, intra-arterially, intrathecally and/or intranasally or directly injected into tissues. For example, the pharmaceutical composition may be administered to patients or subjects by means of infusion or injection. In some embodiments, the administration of the pharmaceutical composition can be done in different ways, such as intravenously, intraperitoneally, subcutaneously, intramuscularly, locally, or intradermally. In some embodiments, the pharmaceutical composition can be administered uninterruptedly. The uninterrupted (or continuous) administration can be achieved by a small pump system worn by the patient to measure the flow of therapeutic agent into the patient, as described in WO2015/036583.

In another aspect, the present application also provides a method for preparing the binding protein, the polypeptide, and the fusion protein of the present application, and the method may include culturing the cell of the present application under a condition enabling the expression of the binding protein of the present application.

In another aspect, the present application provides use of the binding protein, the polypeptide, the fusion protein, the drug molecule, the nucleic acid molecule, the vector, the cell, and/or the pharmaceutical composition in the preparation of a drug for preventing and/or treating a disease and/or disorder.

In another aspect, the present application provides the binding protein, the polypeptide, the fusion protein, the drug molecule, the nucleic acid molecule, the vector, the cell, and/or the pharmaceutical composition, which are used to prevent and/or treat a disease and/or disorder.

In another aspect, the present application provides a method for preventing and/or treating a disease and/or disorder, and the method includes administering to a subject in need thereof an effective amount of the binding protein, the polypeptide, the fusion protein, the drug molecule, the nucleic acid molecule, the vector, the cell, and/or the pharmaceutical composition.

In the present application, the administration can be done in different ways, such as intravenously, intratumorally, intraperitoneally, subcutaneously, intramuscularly, locally, or intradermally.

In some embodiments, the disease and/or disorder includes a tumor.

In some embodiments, the tumor includes a solid tumor and/or a blood tumor.

Without intending to be limited by any theory, the following examples are only to illustrate the binding protein, the preparation method, and the use of the present application, and not intended to limit the scope of the present application.

### EXAMPLES

### Example 1 Molecular construction, Protein expression, Purification and Purity analysis

### 1.1 Molecular construction

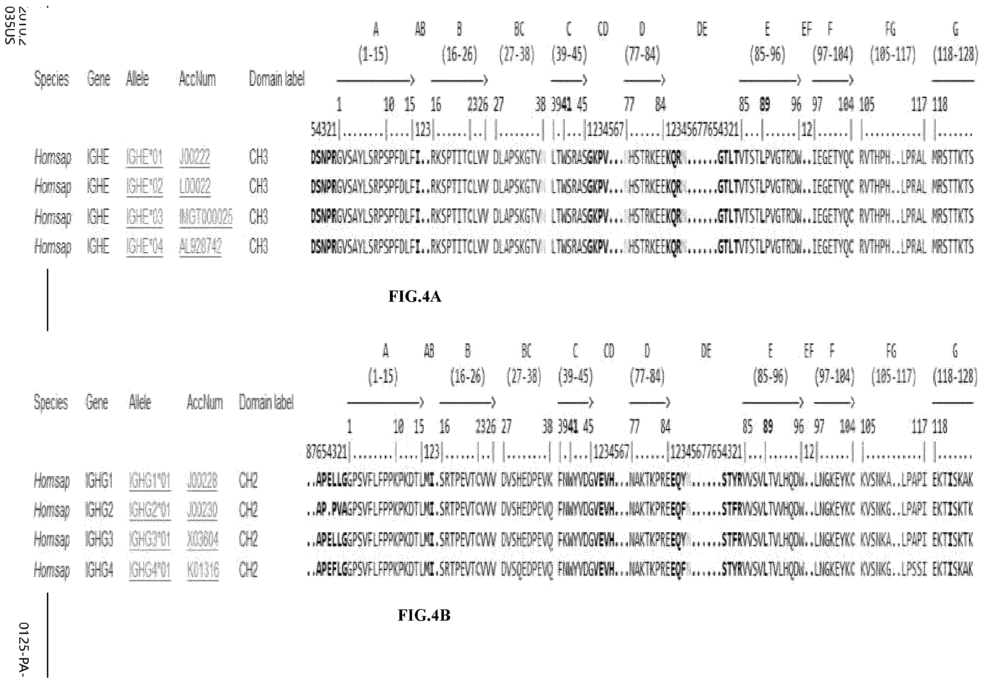

The design of the Fc region of the binding protein was obtained by transplanting the amino acids located in the BC-LOOP, DE-TURN and FG-LOOP regions of the IgE Fc CH3 domain to replace the corresponding amino acids in the BC-LOOP, DE-TURN and FG-LOOP regions of the IgG Fc CH2 domain, or by replacing the amino acids in the BC-LOOP, DE-TURN and FG-LOOP regions of the IgG Fc CH2 domain with part of the amino acids in the BC-LOOP, DE-TURN and FG-LOOP regions located in the IgE Fc CH3 domain. Where, the CH2 in the IgG Fc region after transplant/replacement may include an amino acid sequence as set forth in SEQ ID NO: 11, SEQ ID NO: 41 or SEQ ID NO: 42. The gene encoding the binding protein was synthesized by Genewiz (Suzhou) and Tsingke (Nanjing). For secretory expression in mammalian cells, a signal peptide was added to the N-terminus of the binding protein, with the sequence as set forth below: MGWSCIILFLVATATGVHS (SEQ ID NO: 28). Using standard molecular biology techniques, the gene was cloned into a eukaryotic expression vector (e.g., pCDNA3.1, purchased from Miaoling Bio). After sequencing verification, plasmid preparation was performed using a plasmid Midiprep kit (NucleoBond Xtra Midi Plus, MACHEREY-NAGEL) (see the manufacturer's instructions for specific procedures) for protein expression. Where, FIG. 1 shows the structures of IgE and IgG and a schematic diagram of potential glycosylation modification sites. FIG. 2 shows the structure design of binding proteins and a schematic diagram of potential glycosylation modification sites. The schematic diagram in the figure indicates that a transplant replacement has occurred in the IgG Fc CH2 domain, with some amino acids being replaced with the corresponding amino acids in IgE Fc CH3. According to the structure of binding protein 1 in FIG. 2, the IgG Fc region after transplant replacement may further include the CH2 domain of IgE or a fragment thereof; in FIG. 2, the binding proteins DB177 and DB364 have a structure of binding protein 1; and the binding proteins DB365, DB366, DB-B63 and DB-B64 have a structure of binding protein 2. FIG. 3 shows the structures of binding proteins fused with Fab and a schematic diagram of potential glycosylation modification sites, where, the binding proteins DB871, DB-B229 and DB-B232 have a structure of binding protein 3; and the binding proteins DB872, DB-B230 and DB-B233 have a structure of binding protein 4. FIG. 4A shows the IMGT coding and regional division of the constant region CH3 of a heavy chain of human IgE; FIG. 4B shows the IMGT coding and regional division of the constant region CH2 of a heavy chain of human IgG; and the coding mode is IMGT unique numbering for C-DOMAIN. Where, the amino acid sequence of the binding protein constructed by the present application was shown in Table 1.

### 1.2 Protein expression, purification and purity analysis

Protein expression was achieved by transient transfection of Expi293 cells (purchased from Thermo fisher). The prepared heavy and light chain plasmids were co-transfected in Expi293 cells using PEI-MW40000 (PolySciences) to transiently express the desired proteins. The preparation and use method of PEI can be found in the instructions of PolySciences PEI MAX (also see Jäger, V., et, al. BMC Biotechnol 13, 52, 2013.). The culture and transfection procedures of Expi293 cells can be found in the instructions of Thermofisher Expi293 expression system. 5-7 days after transient transfection, cell supernatants were harvested by centrifugation and the supernatants were filtered through a 0.45 µm filter for subsequent protein purification.

The transiently expressed proteins were purified using a protein A pre-packed column (GE Lifesciences), the procedures for which can be found in the manufacturer's instructions. The harvested eluate protein samples were exchanged by dialysis or ultrafiltration into a PBS buffer system, and then the samples were filtered through a 0.22 µm filter to remove bacteria in order to obtain the protein samples to be tested. The protein samples were quantified using a NanoDrop^{™} spectrophotometer (Thermo Scientific) in combination with the theoretical extinction coefficient of the protein. The results of protein expression and purification showed that the binding proteins were well expressed and could be purified directly using Protein A, retaining similar expression and purification properties of IgG Fc or IgG-like antibodies.

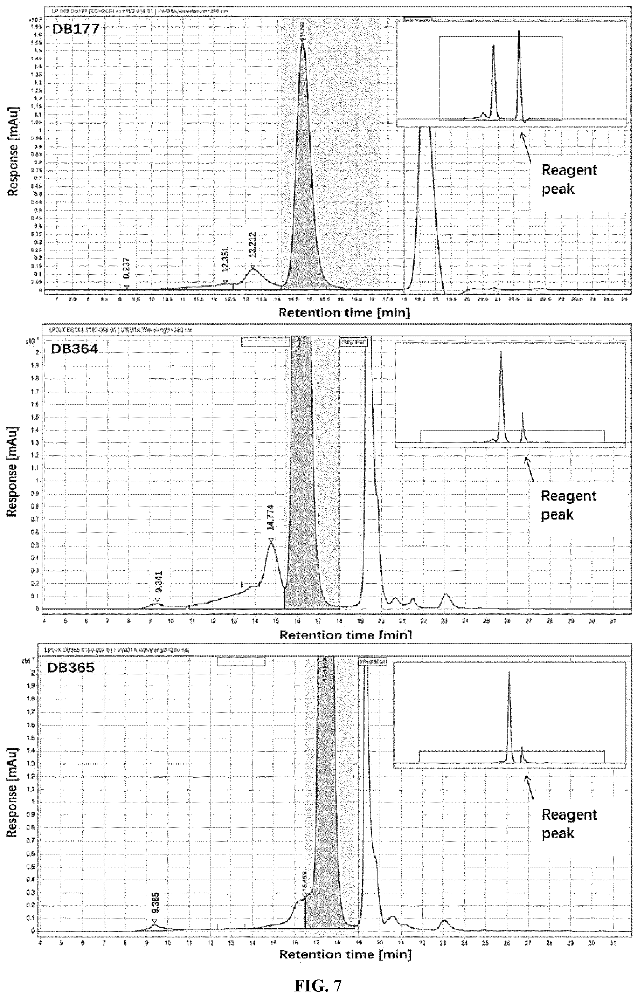

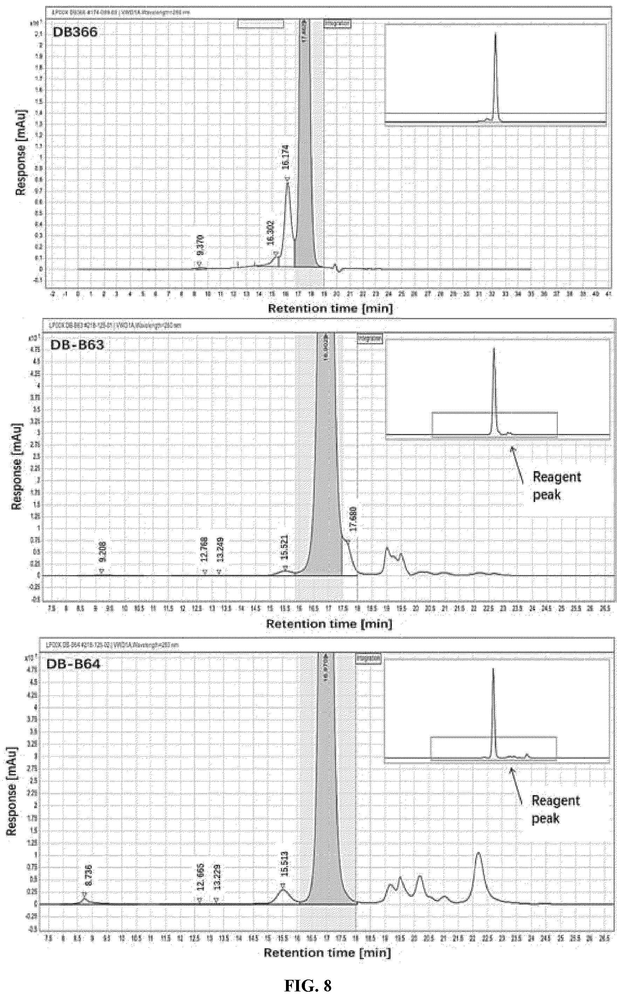

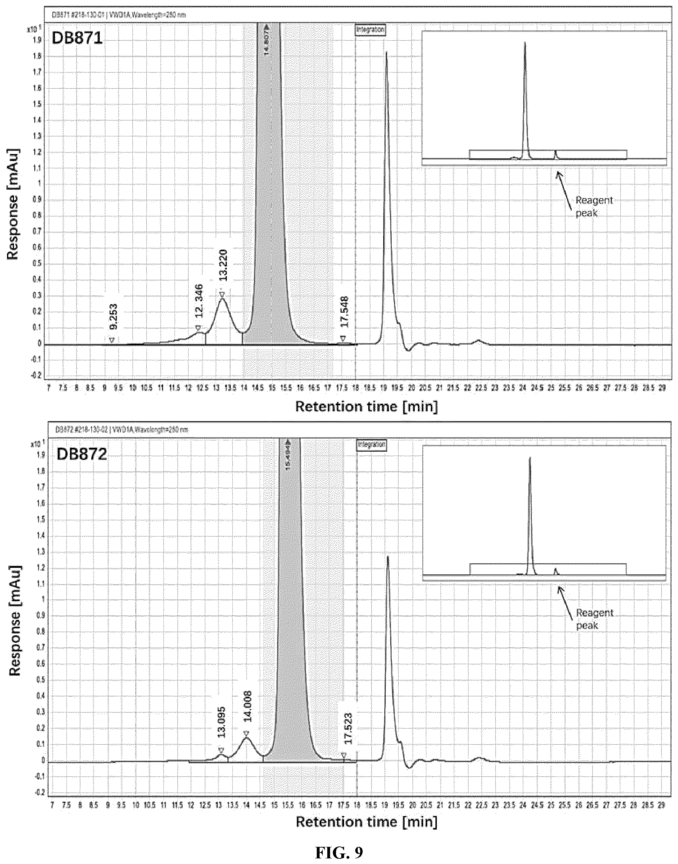

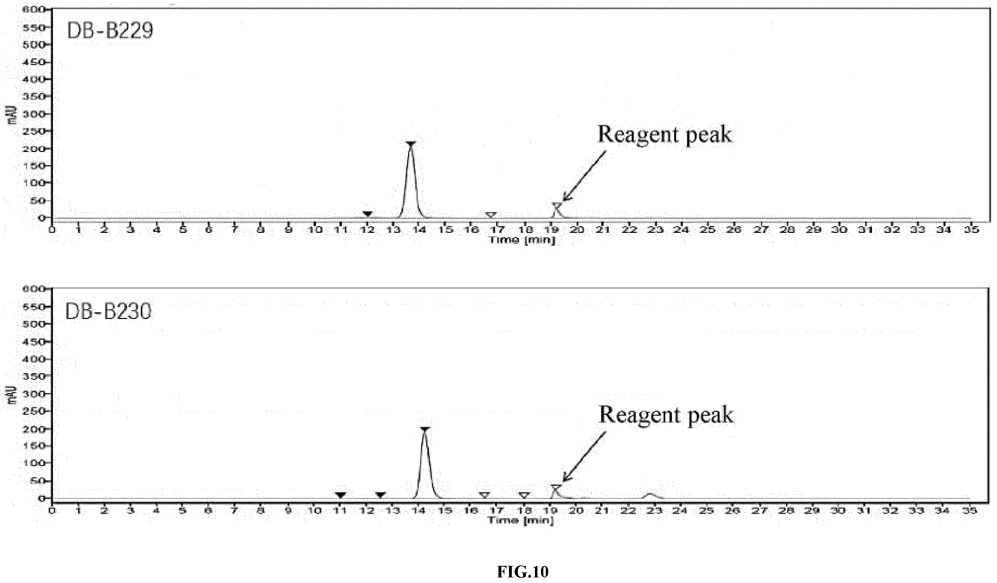

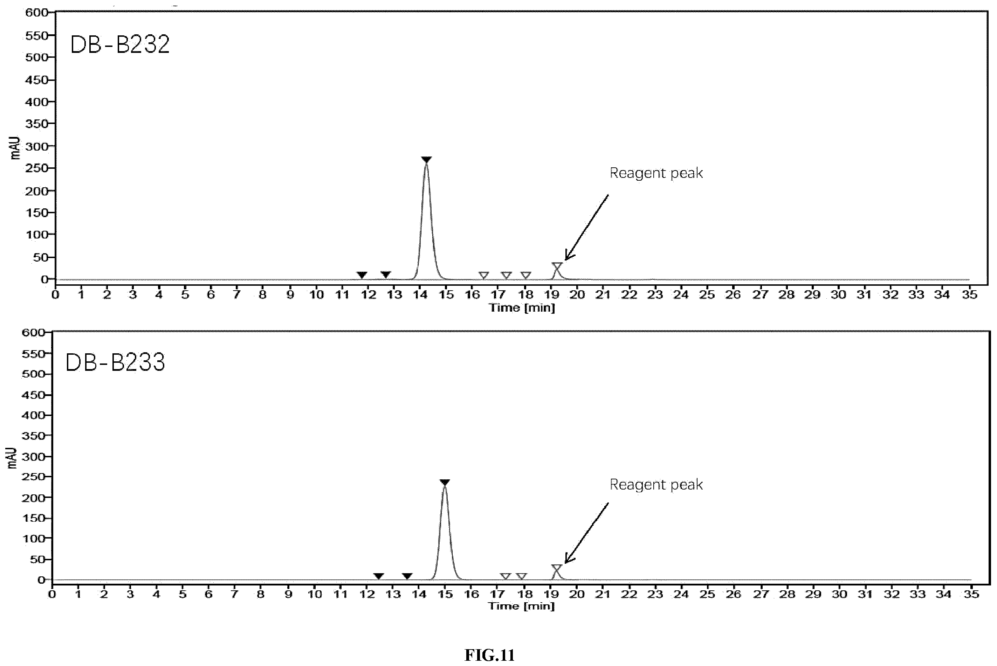

Protein purity was analyzed using size exclusion chromatography (SEC) by applying the purified samples in PBS to a 300 × 4.6 mm, 5 µm column (TOSOH) of TSKgelSuperSW3000. SEC was performed using a U3000-type HPLC instrument (DIONEX). All proteins were determined using UV assay at 280 nm and 214 nm. Elution was performed isocratically at a flow rate of 0.25 mL/min. The analysis results showed that the binding proteins purified in one step using Protein A all had good monomeric purity in the solution state (FIGs. 7-11 and Table 1), where, the purity of DB364 (SEQ ID NO: 17), DB365 (SEQ ID NO: 18) and DB366 (SEQ ID NO: 19) all exceeded 90%, indicating that selective optimization of the sequence of the non-binding region of the binding protein can significantly improve the purity of the protein. And on the basis of DB177 and DB366, the binding proteins DB871 and DB872 fused with Her2 antibody Pertuzumab Fab, the binding proteins DB-B229 and DB-B230 fused with GPC3 antibody Codrituzumab Fab, and the binding proteins DB-B229 and DB-B230 fused with CD20 antibody Rituximab Fab all had a monomeric purity exceeding 95% after purification in one step using Protein A (FIGs. 7-11 and Table 1), indicating that the binding proteins fused with Fab also had excellent purity.

**Table 1 Purity of binding proteins by SEC-HPLC**

| **Protein No.** | **Monomer purity% (SEC)** | **Sequence** |
|---|---|---|
| DB177 | 84.5 | SEQ ID NO: 16 |
| DB364 | 91.9 | SEQ ID NO: 17 |
| DB365 | 97.2 | SEQ ID NO: 18 |
| DB366 | 94.8 | SEQ ID NO: 19 |
| DB-B63 | 96.2 | SEQ ID NO: 30 |
| DB-B64 | 97.2 | SEQ ID NO: 31 |
| DB871 | 97.1 | Heavy chain SEQ ID NO: 20 |
| | | Light chain SEQ ID NO: 22 |
| DB872 | 98.4 | Heavy chain SEQ ID NO: 21 |
| | | Light chain SEQ ID NO: 22 |
| DB-B229 | 97.63 | Heavy chain SEQ ID NO: 32 |
| | | Light chain SEQ ID NO: 34 |
| DB-B230 | 99.11 | Heavy chain SEQ ID NO: 33 |
| | | Light chain SEQ ID NO: 34 |
| DB-B232 | 98.77 | Heavy chain SEQ ID NO: 35 |
| | | Light chain SEQ ID NO: 37 |
| DB-B233 | 99.3 | Heavy chain SEQ ID NO: 36 |
| | | Light chain SEQ ID NO: 37 |

### Example 2 Affinity assay of binding protein to FcεRIa (Bio-layer Interferometry, BLI)

In order to verify the binding ability of the binding proteins to FcεRIa, the Bio-layer Interferometry (BLI, GatorBio) was used to detect the affinity of the binding proteins to FcεRIa. The detailed affinity assay method can be found in the usage or application instructions of the GatorBio instrument, which was briefly described as follows:
the recombinantly expressed FcεRIa-mG2Ahis protein (40 nM, recombinant human FcεRIa extracellular region-mouse IgG2a Fc-6xHis fusion protein, self-produced, protein No. DB967, for the amino acid sequence see SEQ ID NO:) was loaded (loading, 120 s) using an Anti-HIS probe (20-5066, GatorBio), then affinity-bound (Association, 300 s) with a gradient dilution of binding protein at diluted concentrations of 200, 50, 12.5 and 0 (reference line) nM, and then dissociated in a PBST buffer (Dissociation, 500 s), and after the data collection was completed, the affinity was calculated using the software that came with the machine.

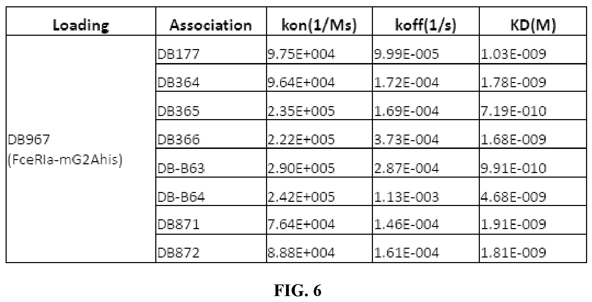

The results of affinity detection showed that all binding proteins could efficiently bind to the recombinant human FcεRIa protein, and the binding ability was relatively consistent, with affinities around 1E-09 M (FIG. 6), indicating that the choice of sequence length of the non-binding region and whether or not to fuse Fab did not affect the affinity. In another aspect, the affinity of the binding protein DB-B64 was slightly reduced, indicating that the amino acids located at positions 1.1-1.6 and 2 of CH2 at the N-terminus of the fusion protein have an effect on the affinity.

### Example 3 Assay of activity of the binding protein to block FcεRIa

To verify the activity of the binding protein to block FcεRIa, the activity of the binding protein to block the binding of FcεRIa to its ligand IgE was detected using ELISA. Specifically, recombinantly expressed human FcεRIa-Fc protein was diluted to 1 µg/mL with PBS, with which the ELISA plate (Corning, Item No.: 42592) was coated at 100 µL/well and incubated at 4°C overnight. On the next day, the coating solution was discarded, and the plate was blocked with PBS containing 2.5% skimmed milk at 25°C for 1 h. After blocking, the plate was washed three times with PBST (PBS + 0.05% Tween 20). Then a premix containing 0.5 nM human IgE or IgE-Fc (biotin-labelled) and a gradient dilution of the binding proteins to be tested (pre-mixed for 1 h) was added and incubated at 25°C for 1 h, where the final concentrations of the binding proteins to be tested were serially diluted 3- or 4-fold starting from 50, 200, or 1200 nM, and with an additional 0 nM point. After incubation, the plate was washed three times with PBST. A 1:2000 dilution of streptavidin-HRP (Sangon Biotech, Shanghai) was added and incubated for 30 min. Unbound secondary antibodies were washed away with PBST buffer. TMB was then added for color development, and the reaction was stopped by adding a stop solution once the color development was completed. The OD₄₅₀ values were measured and analyzed using a four-parameter regression model to calculate the IC₅₀ of the antibody.

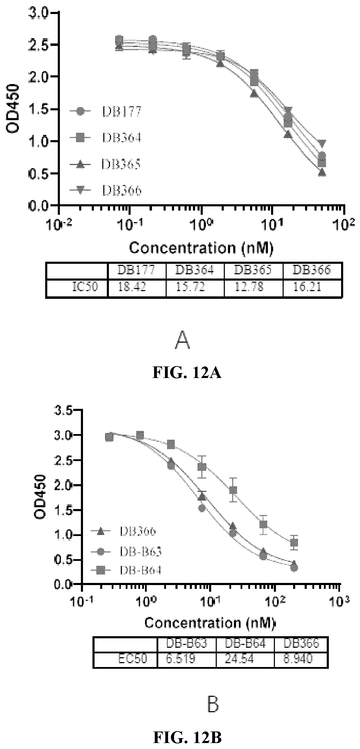

The assay results showed that all binding proteins could effectively block the binding of FcεRIa to its ligand IgE (FIGs. 12A-C), indicating that all binding proteins have blocking activity. In addition, the blocking activities of the binding proteins were compared with DB366 as a reference, and the blocking activities of the binding proteins were basically the same except for DB-B64. Although there were some differences between different groups of experiments, the results of the experiments showed that the blocking activity of DB-B64 was slightly reduced, which were also consistent with the results of the affinity assay.

### Example 4 Assay of activity of the binding protein to activate FcεRI receptors

In order to verify whether the binding proteins have the activity to mediate FcεRI receptor activation, they were detected using the rat mast cell line RBL-2H3 luciferase reporter gene assay.

Construction of human FcεRIα-expressing and NFAT-driven luciferase reporter gene cell lines (RBL-2H3-FcεRIα-NFATLuc cells): based on rat basophil RBL-2H3 cell lines (purchased from Cell Bank of Chinese Academy of Sciences), human FcεRIα-expressing and NFAT-driven luciferase reporter gene cell lines were constructed, which were named as RBL-2H3-FcεRIα-NFATLuc. For the construction process, see: Analytical and Bioanalytical Chemistry volume 412, pages 1901-1914 (2020); Allergy 2010; 65: 1266-1273; J Immunol July 1, 1996, 157 (1) 221-230.

In order to verify whether the binding proteins of the present application have the ability to activate FcεRI receptors, RBL-2H3-FcεRIα-NFATLuc cells were mixed with antigen-expressing cells, and then a gradient dilution of antigen-specific binding proteins was added separately and co-incubated for about 20 hours. After then, luciferase activity assay was carried out to detect the ability of the respective binding proteins to mediate the activation of the FcεRI receptors. Specifically, 40000 RBL-2H3-FcεRIα-NFATLuc cells (25 µL) were mixed uniformly with 4000 antigen-expressing cells (25 µL, Her2-expressing cells were NCI-N87, GPC3-expressing cells were HepG2, and CD20-expressing cells were Raji, all purchased from Cell Bank of Chinese Academy of Sciences) in a 96-well plate, into which were added 50 µL of gradient dilutions of antigen-specific binding proteins (Her2-specific binding proteins DB871 and DB872, 5-fold dilution starting from 5 nM; GPC3-specific binding proteins DB-B229 and DB-B230, 5-fold dilution starting from 10 nM; CD20-specific binding proteins DB-B232 and DB-B233, 5-fold dilution starting from 10 nM), respectively, and co-incubated in an incubator at 37°C and 5% CO₂ for 20 hours, and then luciferase substrates (DD1203-03, Vazyme) were added at 100 µL/well for detection using a microplate reader (Molecular Devices).

The assay results showed that all antigen-specific binding proteins could efficiently mediate FcεRI receptor activation, with EC50s between 16-40 pM (FIGs. 13A-C), with strong activation activity, and it can be seen from the experimental data of DB871 and DB872, DB-B229 and DB-B230, and DB-B232 and DB-B233, that the structural activities of binding proteins 3 and 4 did not differ significantly.

### Example 5 Assay of binding to FcRn

In order to verify whether the binding proteins of the present application retain the ability to bind IgG Fc to FcRn, assays were performed using Bio-layer Interferometry (BLI), with Pertuzumab (see Recommended INN list R51 for specific sequences) as a positive control and human IgE as a negative control. The assay method was briefly described as follows: the recombinantly expressed human FcRn protein (100 nM, with 6xHis tag, FCN-H52W7, ACROBiosystems) was loaded (loading, 120 s) using an Anti-HIS probe (20-5066, GatorBio), then affinity-bound (Association, 300 s) at pH 6.0 buffer conditions with a gradient dilution of control or binding proteins at diluted concentrations of

## Claims

1. A binding protein capable of binding to FcεRI, the binding protein comprises an IgG Fc region or a fragment thereof, the IgG Fc region or the fragment thereof comprises BC-LOOP, DE-TURN and/or FG-LOOP or a fragment thereof derived from a CH3 in an IgE Fc region, and regions in a CH2 in the IgG Fc region corresponding to BC-LOOP, DE-TURN and/or FG-LOOP or the fragment thereof in the CH3 in the IgE Fc region are substituted or replaced by BC-LOOP, DE-TURN and/or FG-LOOP or the fragment thereof in the CH3 in the IgE Fc region.

2. The binding protein according to claim 1, wherein the IgG is a human IgG.

3. The binding protein according to any one of claims 1-2, wherein the IgG is IgG1, IgG2, IgG3 or IgG4.

4. The binding protein according to any one of claims 1-3, wherein a CH2 in a wild-type IgG Fc region comprises an amino acid sequence as set forth in any one of SEQ ID NO: 24, SEQ ID NO: 25, SEQ ID NO: 26, and SEQ ID NO: 27.

5. The binding protein according to any one of claims 1-4, wherein the fragment of the IgG Fc region at least comprises the CH2 in the IgG Fc region or a fragment thereof.

6. The binding protein according to any one of claims 1-5, wherein the BC-LOOP, DE-TURN and/or FG-LOOP or the fragment thereof derived from the CH3 in the IgE Fc region is capable of binding to FcεRI.

7. The binding protein according to any one of claims 1-6, wherein the BC-LOOP in the CH2 in the IgG Fc region comprises an amino acid sequence of positions 27-38 in the CH2 in the IgG Fc region.

8. The binding protein according to any one of claims 1-7, wherein the BC-LOOP in the CH2 in the IgG Fc region comprises an amino acid sequence as set forth in SEQ ID NO: 1 or SEQ ID NO: 2.

9. The binding protein according to any one of claims 1-8, wherein the DE-TURN in the CH2 in the IgG Fc region comprises an amino acid sequence of positions 84.1-84.4 and 85.4-85.1 in the CH2 in the IgG Fc region.

10. The binding protein according to any one of claims 1-9, wherein the DE-TURN in the CH2 in the IgG Fc region comprises an amino acid sequence as set forth in any one of SEQ ID NO: 3, SEQ ID NO: 4, SEQ ID NO: 39, and SEQ ID NO: 40.

11. The binding protein according to any one of claims 1-10, wherein the FG-LOOP in the CH2 in the IgG Fc region comprises an amino acid sequence of positions 105-117 in the CH2 in the IgG Fc region.

12. The binding protein according to any one of claims 1-11, wherein the FG-LOOP in the CH2 in the IgG Fc region comprises an amino acid sequence as set forth in SEQ ID NO: 5, SEQ ID NO: 6 or SEQ ID NO: 7.

13. The binding protein according to any one of claims 1-12, wherein the BC-LOOP in the CH3 in the IgE Fc region comprises an amino acid sequence of positions 27-38 in the CH3 in the IgE Fc region.

14. The binding protein according to any one of claims 1-13, wherein the BC-LOOP in the CH3 in the IgE Fc region comprises an amino acid sequence as set forth in SEQ ID NO: 8.

15. The binding protein according to any one of claims 1-14, wherein the DE-TURN in the CH3 in the IgE Fc region comprises an amino acid sequence of positions 84.1-84.4 and 85.4-85.1 in the CH3 in the IgE Fc region.

16. The binding protein according to any one of claims 1-15, wherein the DE-TURN in the CH3 in the IgE Fc region comprises an amino acid sequence as set forth in SEQ ID NO: 9.

17. The binding protein according to any one of claims 1-16, wherein the FG-LOOP in the CH3 in the IgE Fc region comprises an amino acid sequence of positions 105-117 in the CH3 in the IgE Fc region.

18. The binding protein according to any one of claims 1-17, wherein the FG-LOOP in the CH3 in the IgE Fc region comprises an amino acid sequence as set forth in SEQ ID NO: 10.

19. The binding protein according to any one of claims 1-18, wherein a region location of the amino acid is determined by means of IMGT unique numbering for C-DOMAIN coding.

20. The binding protein according to any one of claims 1-19, wherein the CH2 in the IgG Fc region comprises an amino acid sequence as set forth in SEQ ID NO: 11 or SEQ ID NO: 41.

21. A binding protein, comprising an IgG Fc region or a fragment thereof, compared with a wild-type IgG Fc region, the IgG Fc region or the fragment thereof comprises one or more amino acid mutations, and the mutated IgG Fc region or the fragment thereof is capable of binding to FcεRI.

22. The binding protein according to claim 21, wherein the IgG comprises a human IgG.

23. The binding protein according to any one of claims 21-22, wherein the IgG comprises IgG1, IgG2, IgG3 or IgG4.

24. The binding protein according to any one of claims 21-23, wherein CH2 in the wild-type IgG Fc region comprises an amino acid sequence as set forth in any one of SEQ ID NO: 24, SEQ ID NO: 25, SEQ ID NO: 26, and SEQ ID NO: 27.

25. The binding protein according to any one of claims 21-24, wherein the IgG Fc region at least comprises the CH2 in the IgG Fc region.

26. The binding protein according to claim 25, wherein BC-LOOP in the CH2 in the IgG Fc region has one or more amino acid mutations.

27. The binding protein according to any one of claims 25-26, wherein the BC-LOOP in the CH2 in the IgG Fc region has amino acid mutations at one or more sites selected from a group consisting of: V28, S29, H30, E31, D34, P35, and/or K/Q38.

28. The binding protein according to any one of claims 25-27, wherein the BC-LOOP in the CH2 in the IgG Fc region has one or more amino acid mutations selected from a group consisting of: V28L, S29A, H30P, E31S, D34K, P35G, E36T and/or K/Q38N.

29. The binding protein according to any one of claims 25-28, wherein the BC-LOOP in the CH2 in the mutated IgG Fc region comprises an amino acid sequence as set forth in SEQ ID NO: 8.

30. The binding protein according to any one of claims 25-29, wherein DE-TURN in the CH2 in the IgG Fc region has amino acid mutations at one or more sites selected from a group consisting of: E84.1, Y/F84.3, S85.4, Y/F85.2, and/or R85.1.

31. The binding protein according to any one of claims 25-30, wherein the DE-TURN in the CH2 in the IgG Fc region has one or more amino acid mutations selected from a group consisting of: E84.1K, Y/F84.3R, S85.4G, Y/F85.2L, and/or R85.1T.

32. The binding protein according to any one of claims 25-31, wherein the DE-TURN in the CH2 in the mutated IgG Fc region comprises an amino acid sequence as set forth in SEQ ID NO: 9.

33. The binding protein according to any one of claims 25-32, wherein FG-LOOP in the CH2 in the IgG Fc region has amino acid mutations at one or more sites selected from a group consisting of: K105, S107, N108, K109, A/G110, A/S115, P/S116, and/or I117.

34. The binding protein according to any one of claims 25-33, wherein the FG-LOOP in the CH2 in the IgG Fc region has one or more amino acid mutations selected from a group consisting of: K105R, S107T, N108H, K109P, A/G110H, A/S115R, P/S116A, and/or I117L.

35. The binding protein according to any one of claims 25-34, wherein the FG-LOOP in the CH2 in the mutated IgG Fc region comprises an amino acid sequence as set forth in SEQ ID NO: 10.

36. The binding protein according to any one of claims 1-35, wherein an N terminus of the CH2 in the IgG Fc region has amino acid mutations at one or more sites selected from a group consisting of: P1.5, E1.4, L/P/F1.3, L/V1.2, G/A1.1, G1 and/or P2.

37. The binding protein according to claim 36, wherein the N terminus of the CH2 in the IgG Fc region has one or more amino acid mutations selected from a group consisting of: P1.5D, E1.4S, L/P/F1.3N, L/V1.2P, G/A1.1R and/or P2V.

38. The binding protein according to claim 37, wherein the CH2 in the mutated IgG Fc region comprises an amino acid sequence as set forth in SEQ ID NO: 11 or SEQ ID NO: 41.

39. The binding protein according to any one of claims 21-38, wherein a region location of the amino acid is determined by means of IMGT unique numbering for C-DOMAIN coding.

40. The binding protein according to any one of claims 1-38, further comprising a CH3 in the IgG Fc region.

41. The binding protein according to claim 40, comprising an amino acid sequence as set forth in SEQ ID NO: 29 or SEQ ID NO: 38.

42. The binding protein according to any one of claims 1-41, further comprising a constant region fragment of IgE.

43. The binding protein according to any one of claims 1-42, further comprising CH2 of the constant region of IgE or a fragment thereof.

44. The binding protein according to any one of claims 1-43, wherein the constant region fragment of IgE comprises an amino acid sequence as set forth in any one of SEQ ID NO: 12, SEQ ID NO: 13, SEQ ID NO: 14, and SEQ ID NO: 15.

45. The binding protein according to any one of claims 1-44, comprising an amino acid sequence as set forth in any one of SEQ ID NO: 16, SEQ ID NO: 17, SEQ ID NO: 18, SEQ ID NO: 19, SEQ ID NO: 30, and SEQ ID NO: 31.

46. The binding protein according to any one of claims 1-45, having one or more of the following properties:
(1) being capable of binding to FcεRIa;
(2) being capable of binding to FcRn;
(3) having good expression quality;
(4) being capable of being purified directly with Protein A or G; and
(5) having reduced potential glycosylation sites compared with IgE Fc.

47. A polypeptide, comprising the binding protein according to any one of claims 1-46.

48. A fusion protein, comprising the binding protein according to any one of claims 1-47.

49. The fusion protein according to claim 48, further comprising one or more other functionally active proteins.

50. The fusion protein according to claim 47, wherein the other functionally active proteins are one or more selected from a group consisting of: Fab, scFv, VHH, cytokines, soluble receptors or ligands, and pharmaceutical polypeptides.

51. The fusion protein according to any one of claims 48-50, which is an IgG-like molecule.

52. The fusion protein according to any one of claims 48-51, which is capable of specifically binding to a tumor-associated antigen.

53. The fusion protein according to claim 52, wherein the tumor-associated antigen comprises Her2.

54. The fusion protein according to any one of claims 51-53, comprising a heavy chain, and the heavy chain comprises an amino acid sequence as set forth in SEQ ID NO: 20 or SEQ ID NO: 21.

55. The fusion protein according to any one of claims 51-54, comprising a light chain, and the light chain comprises an amino acid sequence as set forth in SEQ ID NO: 22.

56. The fusion protein according to claim 52, wherein the tumor-associated antigen comprises GPC3.

57. The fusion protein according to claim 56, comprising a heavy chain, and the heavy chain comprises an amino acid sequence as set forth in SEQ ID NO: 32 or SEQ ID NO: 33.

58. The fusion protein according to any one of claims 56-57, comprising a light chain, and the light chain comprises an amino acid sequence as set forth in SEQ ID NO: 34.

59. The fusion protein according to claim 52, wherein the tumor-associated antigen comprises CD20.

60. The fusion protein according to claim 59, comprising a heavy chain, and the heavy chain comprises an amino acid sequence as set forth in SEQ ID NO: 35 or SEQ ID NO: 36.

61. The fusion protein according to any one of claims 59-60, comprising a light chain, and the light chain comprises an amino acid sequence as set forth in SEQ ID NO: 37.

62. A drug molecule, comprising the binding protein according to any one of claims 1-46, the polypeptide according to claim 47 or the fusion protein according to any one of claims 48-61.

63. One or more isolated nucleic acid molecules, encoding the binding protein according to any one of claims 1-46, the polypeptide according to claim 47 or the fusion protein according to any one of claims 48-61.

64. A vector, comprising the nucleic acid molecule according to claim 63.

65. A cell, comprising the nucleic acid molecule according to claim 63 or the vector according to claim 64.

66. A pharmaceutical composition, comprising the binding protein according to any one of claims 1-46, the polypeptide according to claim 47, the fusion protein according to any one of claims 48-61, the drug molecule according to claim 62, the nucleic acid molecule according to claim 63, the vector according to claim 64 or the cell according to claim 65, as well as optionally a pharmaceutically acceptable carrier.

67. A method for preparing the binding protein according to any one of claims 1-46, the polypeptide according to claim 47 or the fusion protein according to any one of claims 48-61, comprising culturing the cell according to claim 59 under a condition enabling the expression of the binding protein, the polypeptide or the fusion protein.

68. Use of the binding protein according to any one of claims 1-46, the polypeptide according to claim 47, the fusion protein according to any one of claims 48-61, the drug molecule according to claim 62, the nucleic acid molecule according to claim 63, the vector according to claim 64, the cell according to claim 65 or the pharmaceutical composition according to claim 66 in preparation of a drug for preventing or treating a disease and/or disorder.

69. The use according to claim 68, wherein the disease and/or disorder comprises a tumor.

70. The use according to any one of claims 68-69, wherein the tumor comprises a solid tumor and/or a blood tumor.
